# EUROPEAN PATENT APPLICATION

(11) **EP 4 324 838 A1**
(43) Date of publication of application: **21.02.2024**
(21) Application number: 22788377.4
(22) Date of filing: 11.04.2022
(51) Int. Cl.: C07H 15/256

(54) **AMPHIPATHIC COMPOUND, PREPARATION METHOD THEREFOR, AND SURFACTANT COMPOSITION COMPRISING SAME**

(30) Priority: 12.04.2021 US 202163173718 P; 28.02.2022 KR 20220025992
(71) Applicant: Pyo, Sang Hyun, Daejeon 35212 (KR)
(72) Inventor: Pyo, Sang Hyun, Daejeon 35212 (KR)
(74) Representative: Rösler Rasch van der Heide & Partner
(86) International application number: PCT/KR2022/005227
(87) International publication number: WO 2022/220521

(57) **Abstract**

The present invention relates to an amphipathic compound, a preparation method therefor, and a surfactant composition comprising same and, specifically, to a compound selected from the group consisting of steviol monoglycoside-acid, steviol monoglycoside-ester, and steviol monoglycoside-amide, a preparation method therefor, and a surfactant composition comprising at least one of these compounds.

## Description

### [Technical Field]

The present invention relates to an amphipathic compound, a method of preparing the same, and a surfactant composition including the same, and more particularly to a compound selected from the group consisting of steviol monoglycoside-acid, steviol monoglycoside-ester, and steviol monoglycoside-amide, a method of preparing the same, and a surfactant composition including at least one selected therefrom.

### [Background Art]

It is necessary to develop new materials capable of minimizing or avoiding environmental pollution and exposure to hazardous chemicals in everyday life. Alkylphenol derivatives, phthalates, and isocyanates are classified as chemicals with serious hazards to the human body and the environment. Thereamong, application of alkylphenols (APs), particularly polyethoxylated derivatives thereof (alkylphenol ethoxylates, APEOs) to commercial surfactants requires more attention. AP mainly serving as a commercial surfactant is nonylphenol (NP), which is primarily used to produce nonylphenol ethoxylate (NPEO) surfactants and is useful in a variety of fields, including paints, latex paints, adhesives, inks, cleaners, pesticide formulations (emulsions), paper industries, textile and leather industries, petroleum recovery chemicals, metal working fluids, cosmetics, detergents, and the like. Metabolites of APEOs are classified as hazardous materials, but these materials are released into the environment through various routes without any special monitoring.

In addition, NP is known to affect humans and animals as an endocrine disruptor. Due to similarity in chemical structure between NP and the female hormone 17β-estradiol, NP may bind to the estrogen receptor instead of 17β-estradiol. Also, for nonionic surfactants (Solutol^{™}, Myrj^{™}, Tween^{™} family, etc.), hydrophilic groups based on polyethylene glycol ((PEG)/polyethylene oxide (PEO)) are attached. These materials are widely useful but have some drawbacks, such as formation of toxic degradation products in aqueous systems (formaldehyde, formic acid, acetaldehyde, etc.), detrimental effects on product stability, chemical instability, and generation of oxidized peroxy radicals.

In addition, most PEG surfactants originate from petrochemicals rather than renewable energy sources, which may be regarded as disadvantageous in view of the impact of surfactants on the environment.

Accordingly, there is a need for more environmentally-friendly and safer alternatives to hazardous APEOs and synthetic surfactants.

### [Disclosure]

### [Technical Problem]

Therefore, the present inventors synthesized a new amphipathic compound serving as a natural environmentally-friendly surfactant.

It is an object of the present invention to provide a new amphipathic compound capable of being used as an environmentally-friendly surfactant and a method of preparing the same.

It is another object of the present invention to provide a surfactant composition including the novel amphipathic compound.

### [Technical Solution]

In order to accomplish the above objects, the present invention provides an amphipathic compound represented by Chemical Formula (1), Chemical Formula (2), or Chemical Formula (3) below.

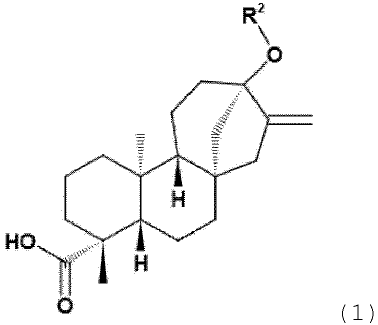

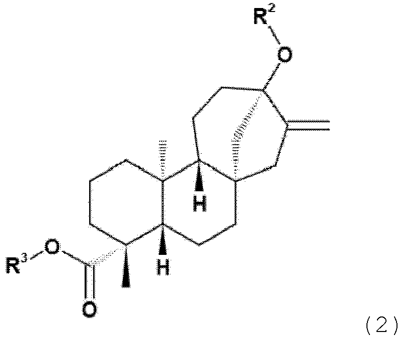

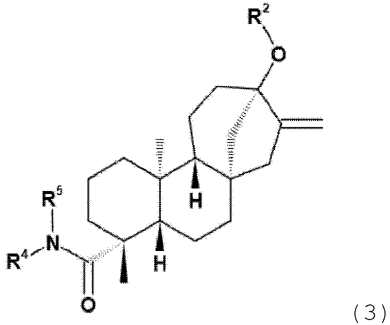

In the above chemical formulas,
R² may be any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1),
R³ may be a linear or cyclic substituted or unsubstituted C₁-C₃₀ alkyl or aryl, and
R⁴ and R⁵ may each independently be a linear or cyclic substituted or unsubstituted C₁-C₃₀ alkyl or aryl, or H.

In an embodiment, any one of R⁴ and R⁵ may be H, and the remaining one may be a linear or cyclic substituted or unsubstituted C₁-C₃₀ alkyl or aryl.

The present invention provides a method of preparing an amphipathic compound of Chemical Formula (1) below, including hydrolyzing glycosyl-ester linkage (-CO-OR¹) of steviol bisglycoside of Chemical Formula (4) below.

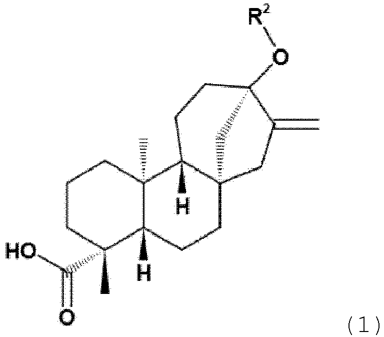

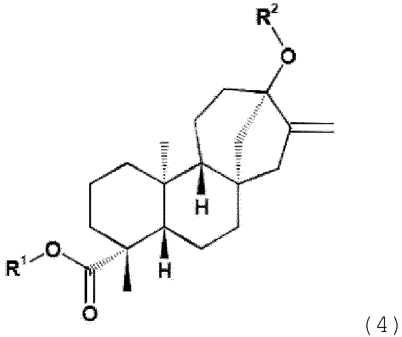

In the above chemical formulas,
R¹ may be β-glucose or β-glucose-β-glucose(2-1), and
R² may be any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1).

In addition, the present invention provides a method of preparing an amphipathic compound of Chemical Formula (2) below, including esterifying steviol monoglycoside-acid of Chemical Formula (1) below with an alkoxy donor.

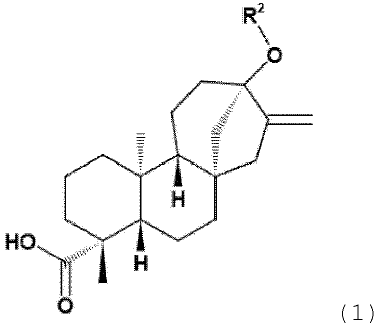

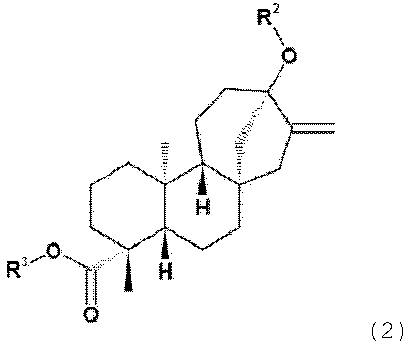

In the above chemical formulas,
R² may be any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1), and
R³ may be a linear or cyclic substituted or unsubstituted C₁-C₃₀ alkyl or aryl.

In addition, the present invention provides a method of preparing an amphipathic compound of Chemical Formula (2) below, including transesterifying steviol bisglycoside of Chemical Formula (4) below with an alkoxy donor.

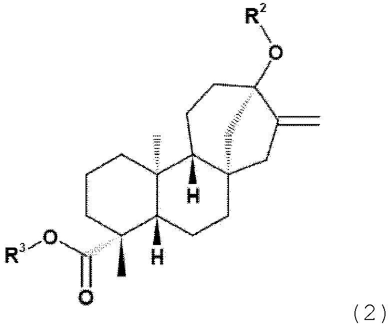

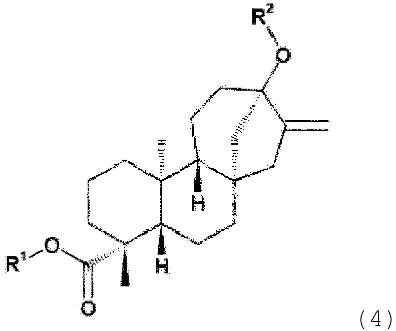

In the above chemical formulas,
R¹ may be β-glucose or β-glucose-β-glucose(2-1),
R² may be any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1), and
R³ may be a linear or cyclic substituted or unsubstituted C₁-C₃₀ alkyl or aryl.

In addition, the present invention provides a method of preparing an amphipathic compound of Chemical Formula (3) below, including amidating steviol monoglycoside-acid of Chemical Formula (1) below with an amine donor.

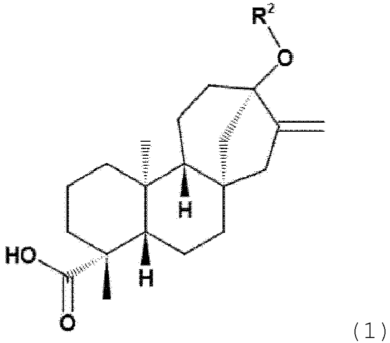

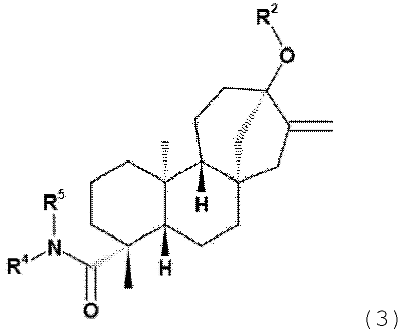

In the above chemical formulas,
R² may be any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1), and
R⁴ and R⁵ may each independently be a linear or cyclic substituted or unsubstituted C₁-C₃₀ alkyl or aryl, or H.

In addition, the present invention provides a method of preparing an amphipathic compound of Chemical Formula (3) below, including transesterifying steviol bisglycoside of Chemical Formula (4) below with an amine donor.

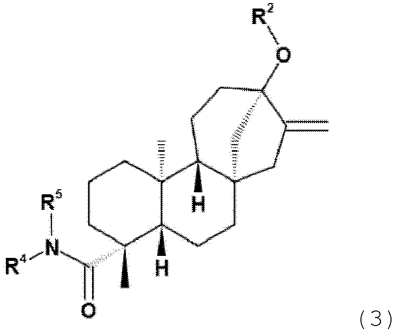

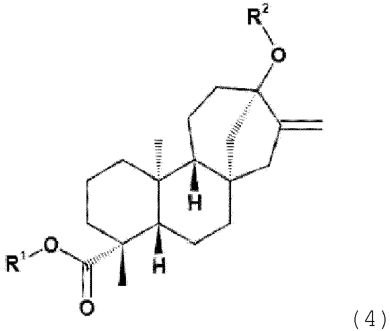

In the above chemical formulas,
R¹ may be β-glucose or β-glucose-β-glucose(2-1),
R² may be any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1), and
R⁴ and R⁵ may each independently be a linear or cyclic substituted or unsubstituted C₁-C₃₀ alkyl or aryl, or H.

The present invention provides a surfactant composition including, as an active ingredient, at least one material selected from the group consisting of steviol monoglycoside-acid, steviol monoglycoside-ester, and steviol monoglycoside-amide.

In an embodiment of the present invention, the steviol monoglycoside-acid may be a compound of Chemical Formula (1) below.

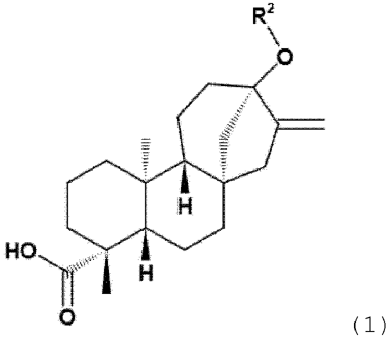

Here, R² may be any one selected from among β-glucose-β-glucose(2-1), β-glucose-β-glucose(2-1)-β-glucose(3-1), β-glucose-β-glucose-β-rhamnose(3-1), and β-glucose-β-xylose(2-1)-β-glucose(3-1).

In an embodiment of the present invention, the steviol monoglycoside-ester may be a compound of Chemical Formula (2) below.

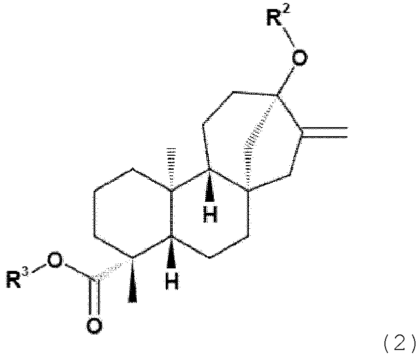

Here, R² may be any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1), and R³ may be a linear or cyclic substituted or unsubstituted C₁-C₃₀ alkyl or aryl.

In an embodiment of the present invention, the steviol monoglycoside-amide may be a compound of Chemical Formula (3) below.

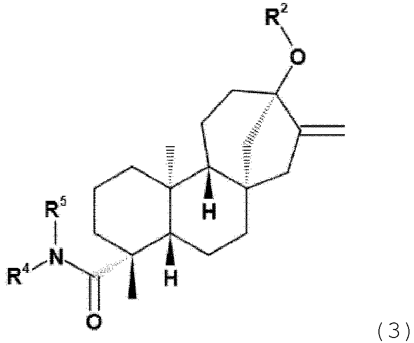

Here, R² may be any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1), and R⁴ and R⁵ may each independently be a linear or cyclic substituted or unsubstituted C₁-C₃₀ alkyl or aryl, or H.

In an embodiment, any one of R⁴ and R⁵ may be H, and the remaining one may be a linear or cyclic substituted or unsubstituted C₁-C₃₀ alkyl or aryl.

### [Advantageous Effects]

According to the present invention, an amphipathic material is constructed to include natural steviol diterpenoid acting as a lipophilic moiety and a hydrophilic glycosyl side chain and thus has the advantage of providing an environmentally-friendly and safe surfactant composition.

### [Mode for Invention]

Surfactants (surface active agents, emulsifiers, detergents) are applied to products requiring increased solubility by decreasing surface tension between two immiscible surfaces. Such a surfactant is amphipathic, with a water-soluble hydrophilic moiety and a lipophilic hydrophobic moiety. Therefore, in an aqueous solution, a soluble surfactant spontaneously forms aggregates (micelles). Here, the hydrophobic moiety faces away from the aqueous phase, i.e. inward, and the hydrophilic moiety faces outward toward the aqueous phase. This is a solubilization process, and the lowest surfactant concentration among surfactant concentration conditions enabling micelle formation is called the critical micelle concentration (CMC).

CMC is one of the important properties of surfactants. At concentrations equal to or greater than CMC, micelles are created when additional surfactants are added. Apart from reaching CMC, surface tension also varies depending on the concentration of the surfactant. After reaching CMC, surface tension remains relatively constant or without great change. CMC directly depends on the structure of the surfactant, and as the length of the alkyl group in the structure increases, CMC decreases and absorption of the surfactant increases. This dependence has direct and important implications for surfactant selection and design. Specifically, the increase in absorption and aggregation ability with an increase in the length of the alkyl group allows the desired effect to be achieved with only a lower concentration of the surfactant. Therefore, combination of a long alkyl group and a long head is usually beneficial to functionality of the surfactant.

The leaves of perennial stevia plants (*Stevia rebaudiana* Bertoni, etc.) contain a high concentration of natural sweetener ingredients. These leaves contain entkaurene-type bisglycosides such as stevioside, rebaudioside A, rebaudioside C, dulcoside A, and the like, which are responsible for a typical sweet taste. Dried stevia leaves generally contain 5-10% stevioside, 2-4% rebaudioside A, and 1-2% rebaudioside C.

This steviol bisglycoside has two hydrophilic glycosyl groups, and use of acid, ester, and amide of steviol monoglycoside having a lipophilic diterpene backbone and one hydrophilic glycosyl group as surfactants is not disclosed.

Steviol monoglycoside obtained from steviol bisglycoside, which is a natural extract, through hydrolysis, transesterification, or transamidation shows very low CMC. For example, CMC of steviol monoglycoside-acid obtained through hydrolysis from rebaudioside A having CMC of 5.14 mM is 1.1 mM, and CMC of steviol monoglycoside-ester obtained through transesterification is 0.15 mM, and CMC of steviol monoglycoside-amide obtained through transamidation is 0.5 mM.

These are biodegradable and biocompatible and thus replace existing hazardous materials and provide an environmentally-friendly approach capable of utilizing abundant plant ingredients.

Various steviol bisglycoside combinations depending on the types of R¹ and R² in Chemical Formula 4 below are shown in Table 1 below.

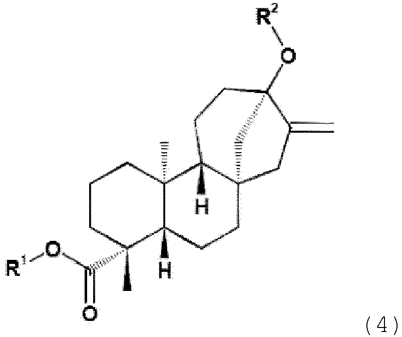

**[Table 1]**

| Product | R¹ | R² |
|---|---|---|
| Stevioside | β-Glucose | β-Glucose-β-Glucose(2-1) |
| Rebaudioside A | β-Glucose | β-Glucose-β-Glucose(2-1)-β-Glucose(3-1) |
| Rebaudioside C | β-Glucose | β-Glucose-β-Glucose(2-1)-β-Rhamnose(3-1) |
| Rebaudioside D | β-Glucose-β-Glucose(2-1) | β-Glucose-β-Glucose(2-1)-β-Glucose(3-1) |
| Rebaudioside E | β-Glucose-β-Glucose(2-1) | β-Glucose-β-Glucose(2-1) |
| Rebaudioside F | β-Glucose | β-Glucose-β-Glucose(2-1)-β-Glucose(3-1) |
| Dulcoside A | β-Glucose | β-Glucose-β-Xylose(2-1)-β-Glucose(3-1) |

In the present invention, it is possible to prepare steviol monoglycoside-acid of Chemical Formula (1) below through hydrolysis of the glycosyl-ester linkage (-CO-OR¹) of steviol bisglycoside.

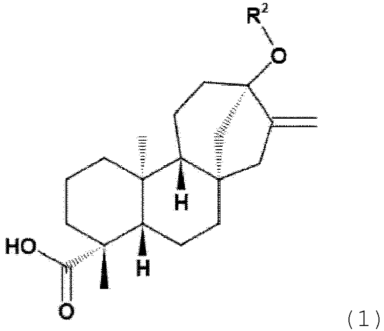

Here, R² may be any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1).

Steviol bisglycoside includes both the extract itself and the mixture. For example, stevioside and rebaudioside E may be converted into steviolbioside through hydrolysis of the glycosyl-ester linkage. An acid or alkali may act as a catalyst for hydrolysis of the glycosyl-ester linkage. The resulting steviol monoglycoside-acid may be used as a surfactant due to amphipathic structure and properties thereof.

In addition, esterification of steviol monoglycoside-acid is capable of preparing steviol monoglycoside-ester of Chemical Formula (2) below by chemical or biological catalysis with alcohol of alkyl and aryl groups.

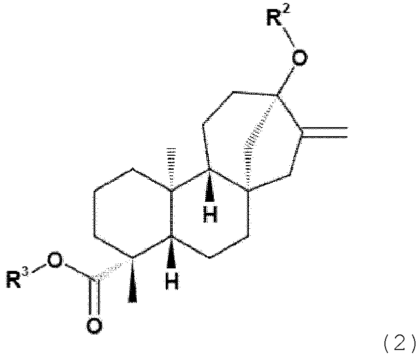

Here, R² may be any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1), and R³ may be a linear or cyclic substituted or unsubstituted C₁-C₃₀ alkyl or aryl.

The resulting steviol monoglycoside-ester may be used as a surfactant due to amphipathic structure and properties thereof.

In addition, amidation of steviol monoglycoside-acid is capable of preparing steviol monoglycoside-amide of Chemical Formula (3) below by chemical or biological catalysis with amine of alkyl and aryl groups.

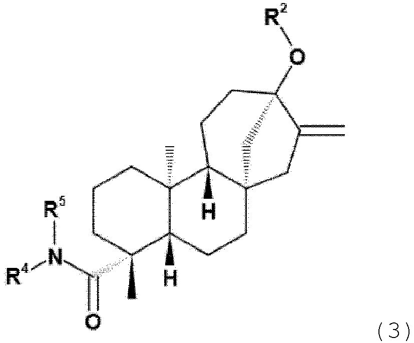

Here, R² may be any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1), and R⁴ and R⁵ may each independently be a linear or cyclic substituted or unsubstituted C₁-C₃₀ alkyl or aryl, or H.

In some cases, any one of R⁴ and R⁵ may be H, and the remaining one may be a linear or cyclic substituted or unsubstituted C₁-C₃₀ alkyl or aryl.

The resulting steviol monoglycoside-amide may be used as a surfactant due to amphipathic structure and properties thereof.

In addition, direct transesterification and amidation of steviol bisglycoside may be carried out by chemical or biological catalysis with alcohol of alkyl and aryl groups and amine of alkyl and aryl groups. The resulting products, namely steviol monoglycoside-ester and steviol monoglycoside-amide, may be used as surfactants in the form of a crude extract or a purified extract.

A better understanding of the present invention may be obtained through the following examples. However, the following examples and test examples are merely set forth to illustrate the present invention, and are not to be construed as limiting the scope of the present invention.

### Synthesis of steviol monoglycoside-ester

### (1) Hydrolysis of steviol bisglycoside and esterification of steviol monoglycoside-acid

### 1) Hydrolysis of steviol bisglycoside by catalysis

The glycosyl-ester linkage of steviol bisglycoside may be hydrolyzed in the presence of an acid or alkali catalyst or by enzyme catalysis to obtain the corresponding acid (almost 100% product selectivity). This reaction may be carried out in water by catalysis. Examples of the acid catalyst may include Bronsted and Lewis acids, such as hydrochloric acid, sulfuric acid, hydrofluoric acid, nitric acid, phosphoric acid, toluenesulfonic acid, polystyrene sulfonate, heteropoly acid, zeolite, nitric acid, silico-aluminate, sulfated zirconia, transition metal oxide, cation exchanger, etc., and examples of the alkali catalyst may include Bronsted and Lewis bases, such as sodium hydroxide, potassium hydroxide, sodium amide, pyridine, imidazole, DBU (1,8-diazabicycloundec-7-ene), guanidine, TBD (1,5,7-triazabicyclo[4.4.0]dec-5-ene), solid base, metal oxide (CaO, BaO, MgO), anion exchanger, etc. Hydrolysis may be carried out in the presence of a catalyst at -20 to 200°C in an aqueous condition or a water-organic solvent mixture system. The organic solvent may be selected from among DMF, DMSO, pyridine, THF, chloroform, dichloromethane, hydrocarbon, cyclic hydrocarbon, alkyl ester, alcohol, ketone, and mixtures thereof, but is not limited thereto. Thereamong, preferred are water-miscible solvents such as DMF, DMSO, pyridine, THF, alcohol (glycol), and mixtures thereof or mixtures therebetween. The organic solvent may be used in an amount of 1 to 3000 vol%, preferably 10 to 1000 vol%, based on the amount of water. The optimal temperature may fall in the range of -20 to 200°C, preferably 10 to 150°C, more preferably 10 to 100°C.

### 2) Esterification of steviol monoglycoside-acid by catalysis

### a) Esterification of steviol monoglycoside-acid by chemical catalysis

Steviol monoglycoside-acid may be esterified with an alkoxy donor such as alcohol in the presence of an acid or alkali catalyst or by enzyme catalysis to obtain the corresponding ester. Examples of the acid catalyst may include Bronsted and Lewis acids, such as hydrochloric acid, sulfuric acid, hydrofluoric acid, nitric acid, phosphoric acid, toluenesulfonic acid, polystyrene sulfonate, heteropoly acid, zeolite, nitric acid, silico-aluminate, sulfated zirconia, transition metal oxide, cation exchanger, etc., and examples of the alkali catalyst may include Bronsted and Lewis bases, such as sodium hydroxide, potassium hydroxide, sodium amide, pyridine, imidazole, DBU (1,8-diazabicycloundec-7-ene), guanidine, TBD (1,5,7-triazabicyclo[4.4.0]dec-5-ene), solid base, metal oxide (CaO, BaO, MgO), anion exchanger, etc. Hydrolysis may be carried out in the presence of a catalyst at -20 to 200°C in an aqueous condition or a water-organic solvent mixture system. The organic solvent may be selected from among DMF, DMSO, pyridine, THF, chloroform, dichloromethane, hydrocarbon, cyclic hydrocarbon, alkyl ester, alcohol, ketone, and mixtures thereof, but is not limited thereto. Thereamong, preferred are water-miscible solvents such as DMF, DMSO, pyridine, THF, alcohol (glycol), and mixtures thereof or mixtures therebetween. The organic solvent may be used in an amount of 1 to 3000 vol%, preferably 10 to 1000 vol%, based on the amount of water. The optimal temperature may fall in the range of -20 to 200°C, preferably 10 to 150°C, more preferably 10 to 100°C.

### b) Esterification of steviol monoglycoside-acid by enzyme

Lipase-catalyzed esterification of steviol monoglycoside-acid is carried out in a solvent system using immobilized *Candida antarctica* lipase B (Novozym^{®}435 (N435)) and an alkoxy donor such as alcohol. Here, the scope of alkoxy donor and enzyme is not limited thereto. The amount of N435 may be 1 to 300 vol%, preferably 5 to 50 vol%, based on the amount of steviol monoglycoside-acid. The organic solvent may be selected from among DMF, DMSO, pyridine, THF, chloroform, dichloromethane, toluene, hydrocarbon, cyclic hydrocarbon, alkyl ester, alcohol, ketone, and mixtures thereof, but is not limited thereto. Thereamong, preferred are DMF, DMSO, pyridine, THF, alcohol (glycol), and mixtures thereof or mixtures therebetween. The organic solvent may be used in an amount of 1 to 3000 vol%, preferably 10 to 1000 vol%, based on the amount of steviol monoglycoside-acid. The alkoxy donor may be used in an amount of 10 to 1000 vol%, preferably 10 to 300 vol%, based on the amount of steviol monoglycoside-acid. A variety of esterases from various sources may be used in this reaction. The optimal temperature for lipase such as *Candida antarctica* lipase B as described in academic literature is 60°C, but may vary depending on the solution used or reaction time. Product selectivity and product yield of the preparation process according to the present invention may also vary depending on conditions.

### (2) Transesterification of steviol bisglycoside by catalysis

### a) Transesterification of steviol bisglycoside by chemical catalysis

Steviol bisglycoside may be transesterified with an alkoxy donor such as alcohol by acid or alkali catalysis to obtain the corresponding ester. This reaction using a catalyst proceeds with alcohol. Examples of the acid catalyst may include Bronsted and Lewis acids, such as hydrochloric acid, sulfuric acid, hydrofluoric acid, nitric acid, phosphoric acid, toluenesulfonic acid, polystyrene sulfonate, heteropoly acid, zeolite, nitric acid, silico-aluminate, sulfated zirconia, transition metal oxide, cation exchanger, etc., and examples of the alkali catalyst may include Bronsted and Lewis bases, such as sodium hydroxide, potassium hydroxide, sodium amide, pyridine, imidazole, DBU (1,8-diazabicycloundec-7-ene), guanidine, TBD (1,5,7-triazabicyclo[4.4.0]dec-5-ene), solid base, metal oxide (CaO, BaO, MgO), anion exchanger, etc. Esterification may be carried out in the presence of a catalyst at -20 to 200°C in an aqueous condition or a water-organic solvent mixture system. The organic solvent may be selected from among DMF, DMSO, pyridine, THF, chloroform, dichloromethane, hydrocarbon, cyclic hydrocarbon, alkyl ester, alcohol, ketone, and mixtures thereof, but is not limited thereto. Thereamong, preferred are water-miscible solvents such as DMF, DMSO, pyridine, THF, alcohol (glycol), and mixtures thereof or mixtures therebetween. The organic solvent may be used in an amount of 1 to 3000 vol%, preferably 10 to 1000 vol%, based on the amount of water. The optimal temperature may fall in the range of -20 to 200°C, preferably 10 to 150°C, more preferably 10 to 100°C.

### b) Transesterification of steviol bisglycoside by enzyme

Lipase-catalyzed transesterification of steviol bisglycoside is carried out in a solvent system using immobilized *Candida antarctica* lipase B (Novozym^{®}435 (N435)) and an alkoxy donor such as alcohol. Here, the scope of alkoxy donor and enzyme is not limited thereto. The amount of N435 may be 1 to 300 vol%, preferably 5 to 50 vol%, based on the amount of steviol bisglycoside. The organic solvent may be selected from among DMF, DMSO, pyridine, THF, chloroform, dichloromethane, toluene, hydrocarbon, cyclic hydrocarbon, alkyl ester, alcohol, ketone, and mixtures thereof, but is not limited thereto. Thereamong, preferred are DMF, DMSO, pyridine, THF, alcohol (glycol), and mixtures thereof or mixtures therebetween. The organic solvent may be used in an amount of 1 to 3000 vol%, preferably 10 to 1000 vol%, based on the amount of steviol monoglycoside-acid. The amine donor may be used in an amount of 10 to 1000 vol%, preferably 10 to 300 vol%, based on the amount of steviol monoglycoside-acid. A variety of esterases from various sources may be used in this reaction. The optimal temperature for lipase such as *Candida antarctica* lipase B as described in academic literature is 60°C, but may vary depending on the solution used or reaction time. Product selectivity and product yield of the preparation process according to the present invention may also vary depending on conditions.

### Synthesis of steviol monoglycoside-amide

### (1) Hydrolysis of steviol bisglycoside and amidation of steviol monoglycoside-acid

### 1) Hydrolysis of steviol bisglycoside by catalysis

The glycosyl-ester linkage of steviol bisglycoside may be hydrolyzed in the presence of an acid or alkali catalyst or by enzyme catalysis to obtain the corresponding acid (almost 100% product selectivity). This reaction may be carried out in water by catalysis. Examples of the acid catalyst may include Bronsted and Lewis acids, such as hydrochloric acid, sulfuric acid, hydrofluoric acid, nitric acid, phosphoric acid, toluenesulfonic acid, polystyrene sulfonate, heteropoly acid, zeolite, nitric acid, silico-aluminate, sulfated zirconia, transition metal oxide, cation exchanger, etc., and examples of the alkali catalyst may include Bronsted and Lewis bases, such as sodium hydroxide, potassium hydroxide, sodium amide, pyridine, imidazole, DBU (1,8-diazabicycloundec-7-ene), guanidine, TBD (1,5,7-triazabicyclo[4.4.0]dec-5-ene), solid base, metal oxide (CaO, BaO, MgO), anion exchanger, etc. Hydrolysis may be carried out in the presence of a catalyst at -20 to 200°C in an aqueous condition or a water-organic solvent mixture system. The organic solvent may be selected from among DMF, DMSO, pyridine, THF, chloroform, dichloromethane, hydrocarbon, cyclic hydrocarbon, alkyl ester, alcohol, ketone, and mixtures thereof, but is not limited thereto. Thereamong, preferred are water-miscible solvents such as DMF, DMSO, pyridine, THF, alcohol (glycol), and mixtures thereof or mixtures therebetween. The organic solvent may be used in an amount of 1 to 3000 vol%, preferably 10 to 1000 vol%, based on the amount of water. The optimal temperature may fall in the range of -20 to 200°C, preferably 10 to 150°C, more preferably 10 to 100°C.

### 2) Amidation of steviol monoglycoside-acid by catalysis

### a) Amidation of steviol monoglycoside-acid by chemical catalysis

Steviol monoglycoside-acid may be amidated with an amine donor such as alkyl or aryl amine in the presence of an acid or alkali catalyst or by enzyme catalysis to obtain the corresponding amide. This reaction may be carried out with amine by catalysis and/or using an activating agent such as imidazole, DBU (1,8-diazabicycloundec-7-ene), guanidine, TBD (1,5,7-triazabicyclo[4.4.0]dec-5-ene), polystyrene-based boronic acid, 2-iodophenylboronic acid, 1,3-bis(2,6-diisopropylphenyl)imidazolium chloride, EDC (1-etyl-3-(3-dimethylaminopropyl)carbodiimide), HATU (1-[bis(dimethylamino)methylene]-1H-1,2,3-triazolo[4,5-b]pyridinium 3-oxide hexafluorophosphate), thionyl chloride, T3P (n-propylphosphonic acid anhydride), CDI (1,1'-carbonyldiimidazole), DCC (dicyclohexylcarbodiimide), thionyl chloride, oxalyl chloride, Ti(OiPr)₄, MnBr(CO)₅, Cp₂ZrCl₂, ZrCl₄, etc. This amidation may be carried out in the presence of a catalyst at -70 to 200°C in an organic solvent, an aqueous condition, or a water-organic solvent mixture system. Here, the organic solvent may be selected from among NMP (N-methyl-2-pyrrolidinone), DMF (N,N-dimethylformamide), DMAc (N,N-dimethylacetamide), DMSO, pyridine, THF, chloroform, dichloromethane, hydrocarbon, cyclic hydrocarbon, alkyl ester, alcohol, ketone, and mixtures thereof, but is not limited thereto. The organic solvent may be used in an amount of 1 to 3000 vol%, preferably 10 to 1000 vol%, based on the amount of water. The optimal temperature may fall in the range of -70 to 200°C, preferably -20 to 150°C, more preferably -20 to 100°C.

### b) Amidation of steviol monoglycoside-acid by enzyme

Lipase-catalyzed amidation of steviol monoglycoside-acid is carried out in a solvent system using immobilized *Candida antarctica* lipase B (Novozym^{®}435 (N435)) and an amine donor such as alkyl or aryl amine. Here, the scope of amine donor and enzyme is not limited thereto. The amount of N435 may be 1 to 300 vol%, preferably 5 to 50 vol%, based on the amount of steviol monoglycoside-acid. The organic solvent may be selected from among NMP (N-methyl-2-pyrrolidinone), DMF (N,N-dimethylformamide), DMAc (N,N-dimethylacetamide), DMSO, pyridine, THF, chloroform, dichloromethane, toluene, hydrocarbon, cyclic hydrocarbon, alkyl ester, alcohol, ketone, and mixtures thereof, but is not limited thereto. Thereamong, preferred are NMP (N-methyl-2-pyrrolidinone), DMF (N,N-dimethylformamide), DMAc (N,N-dimethylacetamide), DMSO, pyridine, THF, alcohol (glycol), and mixtures thereof or mixtures therebetween. The organic solvent may be used in an amount of 1 to 3000 vol%, preferably 10 to 1000 vol%, based on the amount of steviol monoglycoside-acid. The amine donor may be used in an amount of 10 to 1000 vol%, preferably 10 to 300 vol%, based on the amount of steviol monoglycoside-acid. A variety of amidases from various sources may be used in this reaction. The optimal temperature for lipase such as *Candida antarctica* lipase B as described in academic literature is 60°C, but may vary depending on the solution used or reaction time. Product selectivity and product yield of the preparation process according to the present invention may also vary depending on conditions.

### (2) Transamidation of steviol bisglycoside by catalysis

### a) Transamidation of steviol bisglycoside by chemical catalysis

Steviol bisglycoside may be amidated with an amine donor such as alkyl or aryl amine in the presence of an acid or alkali catalyst or by enzyme catalysis to obtain the corresponding amide. This reaction may be carried out with amine by catalysis and/or using an activating agent such as imidazole, DBU (1,8-diazabicycloundec-7-ene), guanidine, TBD (1,5,7-triazabicyclo[4.4.0]dec-5-ene), polystyrene-based boronic acid, 2-iodophenylboronic acid, 1,3-bis(2,6-diisopropylphenyl)imidazolium chloride, EDC (1-etyl-3-(3-dimethylaminopropyl)carbodiimide), HATU (1-[bis(dimethylamino)methylene]-1H-1,2,3-triazolo[4,5-b]pyridinium 3-oxide hexafluorophosphate), thionyl chloride, T3P (n-propylphosphonic acid anhydride), CDI (1,1'-carbonyldiimidazole), DCC (dicyclohexylcarbodiimide), thionyl chloride, oxalyl chloride, Ti(OiPr)₄, MnBr(CO)₅, Cp₂ZrCl₂, ZrCl₄, etc. Examples of the acid catalyst may include Bronsted and Lewis acids, such as hydrochloric acid, sulfuric acid, hydrofluoric acid, nitric acid, phosphoric acid, toluenesulfonic acid, polystyrene sulfonate, heteropoly acid, zeolite, nitric acid, silico-aluminate, sulfated zirconia, transition metal oxide, cation exchanger, etc., and examples of the alkali catalyst may include Bronsted and Lewis bases, such as sodium hydroxide, potassium hydroxide, sodium amide, pyridine, imidazole, DBU (1,8-diazabicycloundec-7-ene), guanidine, TBD (1,5,7-triazabicyclo[4.4.0]dec-5-ene), solid base, metal oxide (CaO, BaO, MgO), anion exchanger, etc. This amidation may be carried out in the presence of a catalyst at -70 to 200°C in an organic solvent, an aqueous condition, or a water-organic solvent mixture system. Here, the organic solvent may be selected from among NMP (N-methyl-2-pyrrolidinone), DMF (N,N-dimethylformamide), DMAc (N,N-dimethylacetamide), DMSO, pyridine, THF, chloroform, dichloromethane, hydrocarbon, cyclic hydrocarbon, alkyl ester, alcohol, ketone, and mixtures thereof, but is not limited thereto. The organic solvent may be used in an amount of 1 to 3000 vol%, preferably 10 to 1000 vol%, based on the amount of water. The optimal temperature may fall in the range of - 70 to 200°C, preferably -20 to 150°C, more preferably -20 to 100°C.

### b) Transamidation of steviol bisglycoside by enzyme

Lipase-catalyzed amidation of steviol bisglycoside is carried out in a solvent system using immobilized *Candida antarctica* lipase B (Novozym^{®}435 (N435)) and an amine donor such as alkyl or aryl amine. Here, the scope of amine donor and enzyme is not limited thereto. The amount of N435 may be 1 to 300 vol%, preferably 5 to 50 vol%, based on the amount of steviol bisglycoside. The organic solvent may be selected from among NMP (N-methyl-2-pyrrolidinone), DMF (N,N-dimethylformamide), DMAc (N,N-dimethylacetamide), DMSO, pyridine, THF, chloroform, dichloromethane, toluene, hydrocarbon, cyclic hydrocarbon, alkyl ester, alcohol, ketone, and mixtures thereof, but is not limited thereto. Thereamong, preferred are NMP (N-methyl-2-pyrrolidinone), DMF (N,N-dimethylformamide), DMAc (N,N-dimethylacetamide), DMSO, pyridine, THF, alcohol (glycol), and mixtures thereof or mixtures therebetween. The organic solvent may be used in an amount of 1 to 3000 vol%, preferably 10 to 1000 vol%, based on the amount of steviol monoglycoside-acid. The amine donor may be used in an amount of 10 to 1000 vol%, preferably 10 to 300 vol%, based on the amount of steviol monoglycoside-acid. A variety of amidases from various sources may be used in this reaction. The optimal temperature for lipase such as *Candida antarctica* lipase B as described in academic literature is 60°C, but may vary depending on the solution used or reaction time. Product selectivity and product yield of the preparation process according to the present invention may also vary depending on conditions.

### <Example 1> Hydrolysis of stevioside by alkali catalysis

402.5 mg (0.5 mmol) of stevioside was added to 12.5 ml of a NaOH solution (1.0 g of NaOH/12.5 ml of methanol) and allowed to react at 60°C for 5 hours. The reaction solution was neutralized with 1 N HCl at room temperature and extracted three times with n-butanol. The organic layer was collected, washed with water, and distilled under reduced pressure to obtain a crude solid product. This crude solid product was recrystallized from a 1:1 mixture of methanol and acetone to obtain steviolbioside (273 mg, 85% (mol/mol) yield) as purified steviol monoglycoside-acid. Conversion of the glucosyl ester linkage into the corresponding acid through hydrolysis was confirmed by FT-IR and NMR. After hydrolysis of stevioside, the strong peak of carbonyl (C=O) observed in FT-IR shifted from 1640 cm⁻¹ (glucosyl ester) to 1731 cm⁻¹ (acid). The molecular weight of the product was measured as m/z 642.72[M]⁺ (calculated for C₃₂H₅₀O₁₃, 642.73).

### <Example 2> Esterification of steviol monoglycoside-acid by alkali catalysis

An alkali catalyst solution (phosphine/I₂/base) was prepared by adding I₂ (0.15 mmol) to 2 ml of CH₂Cl₂ and further adding imidazole (0.33 mmol) thereto. 64.3 mg (0.1 mmol) of the product obtained in Example 1 was added to the catalyst solution and mixed at room temperature for 5 minutes, after which ethanol (0.15 mmol) was added thereto. After reaction for 15 hours, 2 ml of CH₂Cl₂ was added thereto, followed by washing with 2 N HCl and water and drying using anhydrous Na₂SO₄ to obtain a crude solid product. Thereafter, CH₂Cl₂ was evaporated under reduced pressure and silica chromatography (ethyl acetate/n-hexane mixed solution) was performed to obtain purified steviol glycoside-ester (ethyl) (52.3 mg, 78% (mol/mol) yield). Conversion of the acid into the corresponding ethylester through esterification was confirmed by FT-IR and NMR. After esterification of steviolbioside, the strong peak of carbonyl (C=O) observed in FT-IR shifted from 1728 cm⁻¹ (acid) to 1715 cm⁻¹ (ester) . Esterification of ethanol was confirmed through a new peak (-CO-OCH₂-, 3.85 ppm, 2H, q) in ¹H NMR (400 MHz, CDCl₃). The molecular weight of the product was measured as m/z 670.75[M]⁺ (calculated for C₃₄H₅₄O₁₃, 670.78).

### <Example 3> Amidation of steviol monoglycoside-acid by alkali catalysis

An alkali catalyst solution (phosphine/I₂/base) was prepared by adding I₂ (0.15 mmol) to 2 ml of CH₂Cl₂ and further adding imidazole (0.33 mmol) thereto. 64.3 mg (0.1 mmol) of the product obtained in Example 1 was added to the catalyst solution and mixed at room temperature for 5 minutes, after which n-octylamine (0.15 mmol) was added thereto. After reaction for 15 hours, 2 ml of CH₂Cl₂ was added thereto, followed by washing with 2 N HCl and water and drying using anhydrous Na₂SO₄ to obtain a crude solid product. Thereafter, CH₂Cl₂ was evaporated under reduced pressure and silica chromatography (ethyl acetate/n-hexane mixed solution) was performed to obtain purified steviol glycoside-amide (octyl) (56.5 mg, 75% (mol/mol) yield). Conversion of the acid into the corresponding octyl-amide through amidation was confirmed by FT-IR and NMR. After amidation of steviolbioside, the strong peak of carbonyl (C=O) observed in FT-IR shifted from 1728 cm⁻¹ (acid) to 1645 cm⁻¹ (amide). The molecular weight of the product was measured as m/z 754.12[M]⁺ (calculated for C₄₀H₆₇O₁₂N, 753.96).

### <Example 4> Direct transesterification of rebaudioside A by acid catalysis

484 mg (0.5 mmol) of rebaudioside A and 12 mg of p-toluenesulfonic acid were added to 5 ml of methanol, allowed to react at 60°C for 12 hours, concentrated through evaporation under reduced pressure, and extracted three times using n-butanol in water. The organic layer was collected, washed with brine, and evaporated under reduced pressure to obtain 392 mg (95.7% yield) of a product. After esterification of rebaudioside A, the strong peak of carbonyl (C=O) observed in FT-IR shifted from 1640 cm⁻¹ (glucosyl ester) to 1716 cm⁻¹ (ester). The molecular weight of the product was measured as m/z 819.3[M]⁺ (calculated for C₃₉H₆₂O₁₈, 818.9).

### <Example 5> Direct transamidation of rebaudioside A by acid catalysis

484 mg (0.5 mmol) of rebaudioside A and 12 mg of p-toluenesulfonic acid were added to 5 ml of n-hexylamine, allowed to react at 60°C for 12 hours, concentrated through evaporation under reduced pressure, and extracted three times using n-butanol in water. The organic layer was collected, washed with brine, and evaporated under reduced pressure to obtain a crude solid product. This crude solid product was recrystallized from a 1:1 mixture of methanol and acetone to obtain 350 mg (78.8% yield) of a product. After amidation of rebaudioside A, the strong peak of carbonyl (C=O) observed in FT-IR shifted from 1640 cm⁻¹ (glucosyl ester) to 1645 cm⁻¹ (amide). The molecular weight of the product was measured as m/z 888.24[M]⁺ (calculated for C₄₄H₇₃O₁₇N, 888.04).

### <Test Example 1> Measurement of HLB value

The HLB (hydrophile-lipophile balance) value of a product was determined using a Griffin's calculation method. HLB values may vary depending on glycosyl, ester, and amide groups.

Respective HLB values of the products of Examples 1 to 3 calculated based on the hydrophilic group and molecular weight were 10.6, 10.2, and 9.1. Here, hydration properties thereof may be predicted from the HLB values. The HLB value may be adjusted using various alcohol and amine side chains as lipophilic groups.

### <Test Example 2> Measurement of CMC

### CMC (critical micelle concentration)

The surface tension values of steviol bisglycoside (rebaudioside A) and steviol monoglycoside-acid, ester, and amide obtained in Examples 1, 4, and 5 were measured and compared at various concentrations using the stalagmometric method. In addition, through a graph of surface tension versus sample concentration, CMC was measured to be 5.14 mM (rebaudioside A), 1.1 mM (Example 1, steviolbioside), 0.15 mM (Example 4, rebaudioside B methyl ester), and 0.5 mM (Example 5, rebaudioside B n-hexylamide). This shows that the CMC of the products obtained through hydrolysis, (trans)esterification, and (trans)amidation is greatly decreased compared to steviol bisglycoside.

### <Test Example 3> Analysis of surfactant properties

The foaming properties of steviol monoglycoside-ester obtained in Example 4 were confirmed. 5 ml of distilled water and 10 mg of steviol monoglycoside-ester obtained in Example 4 were mixed in a measuring cylinder to form micelles, then shaken for 10 seconds to generate foam, then allowed to stand at room temperature to compare the amount of foam.

Thereby, it was confirmed that about 50% of the foam was maintained after 15 minutes and about 30% of the foam was maintained after 30 minutes, based on the height of the foam.

As described hereinbefore, preferred embodiments of the present invention have been described with reference to the drawings, but those skilled in the art will understand that various modifications and alterations can be made to the present invention without departing from the spirit and scope of the present invention as set forth in the claims below.

## Claims

1. An amphipathic compound represented by Chemical Formula (1), Chemical Formula (2), or Chemical Formula (3) below: 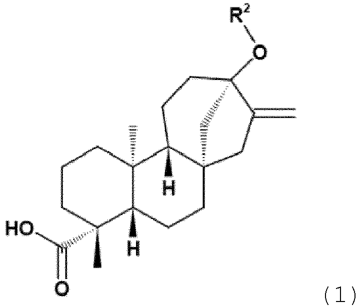 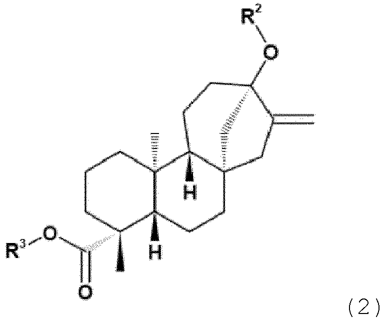 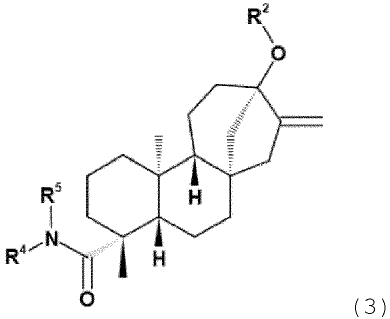 wherein:
R² is any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1);
R³ is a linear or cyclic substituted or unsubstituted C₁-C₃₀ alkyl or aryl; and
R⁴ and R⁵ are each independently a linear or cyclic substituted or unsubstituted C₁-C₃₀ alkyl or aryl, or H.

2. The amphipathic compound according to claim 1, wherein any one of R⁴ and R⁵ is H, and a remaining one is a linear or cyclic substituted or unsubstituted C₁-C₃₀ alkyl or aryl.

3. A method of preparing an amphipathic compound of Chemical Formula (1) below, comprising hydrolyzing glycosyl-ester linkage (-CO-OR¹) of steviol bisglycoside of Chemical Formula (4) below: 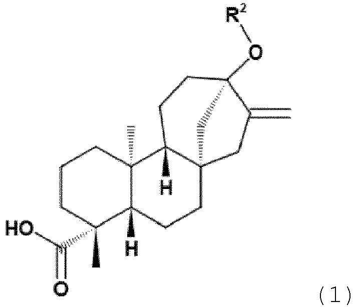 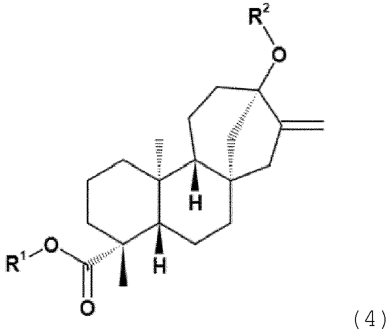 wherein:
R¹ is β-glucose or β-glucose-β-glucose(2-1); and
R² is any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1).

4. A method of preparing an amphipathic compound of Chemical Formula (2) below, comprising esterifying steviol monoglycoside-acid of Chemical Formula (1) below with an alkoxy donor: 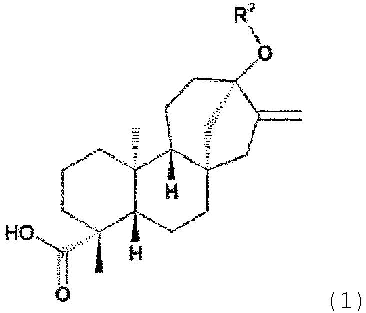 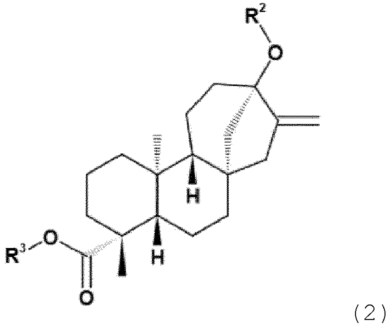 wherein:
R² is any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1); and
R³ is a linear or cyclic substituted or unsubstituted C₁-C₃₀ alkyl or aryl.

5. A method of preparing an amphipathic compound of Chemical Formula (2) below, comprising transesterifying steviol bisglycoside of Chemical Formula (4) below with an alkoxy donor: 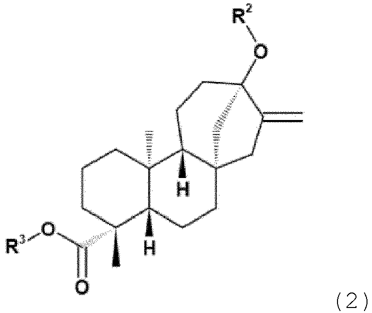 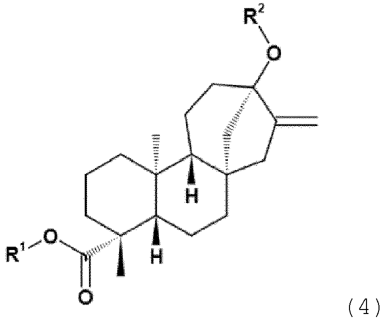 wherein:
R¹ is β-glucose or β-glucose-β-glucose(2-1);
R² is any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1); and
R³ is a linear or cyclic substituted or unsubstituted C₁-C₃₀ alkyl or aryl.

6. A method of preparing an amphipathic compound of Chemical Formula (3) below, comprising amidating steviol monoglycoside-acid of Chemical Formula (1) below with an amine donor: 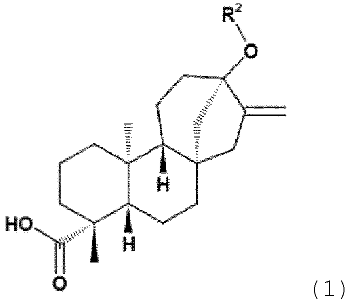 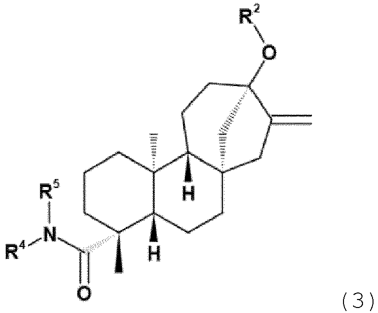 wherein:
R² is any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1); and
R⁴ and R⁵ are each independently a linear or cyclic substituted or unsubstituted C₁-C₃₀ alkyl or aryl, or H.

7. A method of preparing an amphipathic compound of Chemical Formula (3) below, comprising transesterifying steviol bisglycoside of Chemical Formula (4) below with an amine donor: 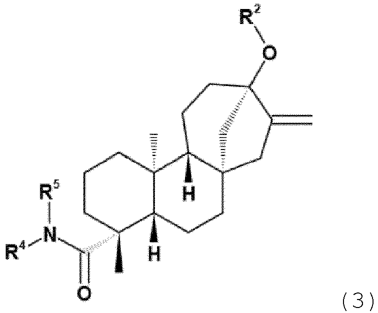 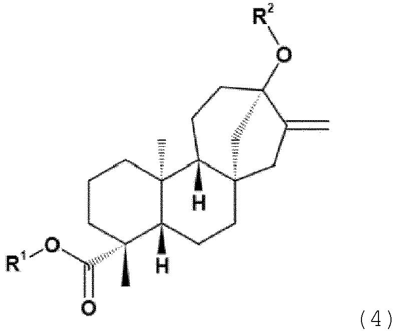 wherein:
R¹ is β-glucose or β-glucose-β-glucose(2-1);
R² is any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1); and
R⁴ and R⁵ are each independently a linear or cyclic substituted or unsubstituted C₁-C₃₀ alkyl or aryl, or H.

8. A surfactant composition comprising, as an active ingredient, at least one material selected from the group consisting of steviol monoglycoside-acid, steviol monoglycoside-ester, and steviol monoglycoside-amide.

9. The surfactant composition according to claim 1, wherein the steviol monoglycoside-acid is a compound of Chemical Formula (1) below: 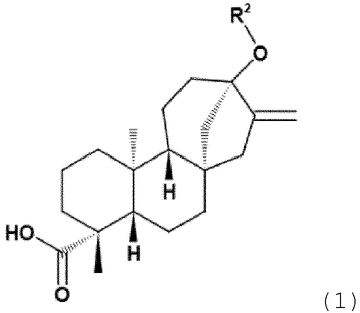 wherein R² is any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1).

10. The surfactant composition according to claim 1, wherein the steviol monoglycoside-ester is a compound of Chemical Formula (2) below: 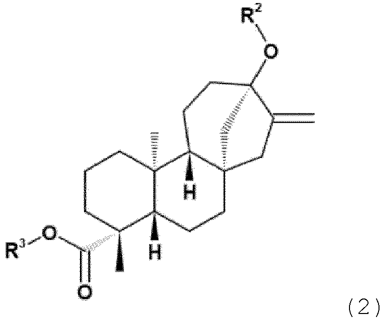 wherein R² is any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1), and R³ is a linear or cyclic substituted or unsubstituted C₁-C₃₀ alkyl or aryl.

11. The surfactant composition according to claim 1, wherein the steviol monoglycoside-amide is a compound of Chemical Formula (3) below: 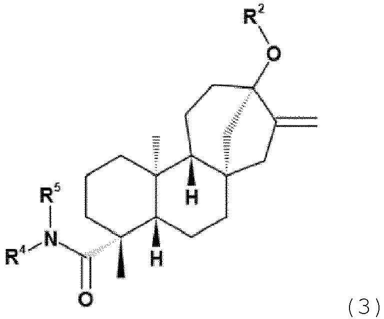 wherein R² is any one selected from among β-glucose-β-glucose(2-1), β-glucose-β-glucose(2-1)-β-glucose(3-1), β-glucose-β-glucose(2-1)-β-rhamnose(3-1), and β-glucose-β-xylose(2-1)-β-glucose(3-1), and R⁴ and R⁵ are each independently a linear or cyclic substituted or unsubstituted C₁-C₃₀ alkyl or aryl, or H.

12. The surfactant composition according to claim 4, wherein any one of R⁴ and R⁵ is H, and a remaining one is a linear or cyclic substituted or unsubstituted C₁-C₃₀ alkyl or aryl.
